# EUROPÄISCHE PATENTSCHRIFT

(11) **EP 1 124 853 B1**
(45) Veröffentlichungstag und Bekanntmachung des Hinweises auf die Patenterteilung: **17.05.2006**
(21) Anmeldenummer: 99962045.3
(22) Anmeldetag: 01.11.1999
(51) Int. Cl.: C07K 16/00, C07K 16/44, C07K 16/16, G01N 33/53

(54) **ANTI-PETASIN-ANTIKÖRPER, VERFAHREN ZU IHRER HERSTELLUNG UND IHRE VERWENDUNG**
ANTI-PETASIN ANTIBODIES, METHODS FOR THE PRODUCTION THEREOF AND THEIR USE
ANTICORPS CONTRE LA PETASINE, LEURS PROCEDES DE PRODUCTION ET LEUR UTILISATION

(30) Priorität: 30.10.1998 DE 19850011; 30.11.1998 DE 19856777
(43) Veröffentlichungstag der Anmeldung: 22.08.2001
(73) Patentinhaber: Max Zeller Söhne AG, 8590 Romanshorn (CH)
(72) Erfinder: SCHÖSSLER, Werner, D-16341 Schwanebeck (DE); HENTSCHEL, Christian, D-16356 Birkholz (DE); TACK, Vivianne, D-60431 Frankfurt am Main (DE)
(74) Vertreter: Baumbach, Friedrich
(86) Internationale Anmeldenummer: PCT/DE1999/003525
(87) Internationale Veröffentlichungsnummer: WO 2000/026255

(56) Entgegenhaltungen:
- EP-A- 0 392 976
- EP-A- 0 645 628
- WITSCHEL M C ET AL: "Synthese der Pestwurzinhaltsstoffe (+)-Petasin und (+)-Isopetasin" TETRAHEDRON LETTERS,NL,ELSEVIER SCIENCE PUBLISHERS, AMSTERDAM, vol. 36, no. 19, 8 May 1995 (1995-05-08), pages 3325-3328, XP004028122 ISSN: 0040-4039
- PATENT ABSTRACTS OF JAPAN vol. 014, no. 057 (C-0684), 2 February 1990 (1990-02-02) & JP 01 283218 A (MATSUDAIRA TENNENBUTSU KENKYUSHO:KK;OTHERS: 01), 14 November 1989 (1989-11-14)
- LANGER T ET AL.: "A competitive enzame immunoassay for the pyrralizidine alkaloids of the senecionine type" PLANTA MEDICA, vol. 62, no. 3, June 1996 (1996-06), pages 267-71, XP000877221

## Beschreibung

Die Erfindung betrifft Anti-Petasin-Antikörper zum in vitro Nachweis von Petasin oder Petasin-Protein-Konjugaten in physiologischen Flüssigkeiten, die keine Kreuzreaktivität gegenüber Strukturanaloga oder Metaboliten des Petasins bzw. gegenüber nicht zur Immunisierung eingesetzten Derivaten des Petasins besitzen, Verfahren zu ihrer Herstellung mittels Immunisierung durch Carrier-Molekül-gekoppelte Petasin-Derivate, sowie ihre Verwendung und einen Testkit.

Petasin, eine Komponente von Pestwurzextrakten, ist bekanntermaßen der Ester aus Petasol und Angelikasäure, der schon länger als pflanzliches Spasmoanalgetikum zur Bekämpfung von Spasmen des Gastrointestinaltraktes, insbesondere Harnleiterkoliken, spastischen Bronchitiden und Migräne, sowie antiphlogistisch verwendet wird (B. Debrunner et al., Pharm. Acta Helv. 72, 359-380 (1998). Weiterhin schreibt man Petasindrogen eine Antitumorwirkung zu (B. Meier et al., Hagers Handbuch der pharmazeutischen Praxis, 5. Auflage, S. 81-105, Springer-Verlag (1994)). Inzwischen liegen auch neuere Erkenntnisse zur Beeinflussung der Biosynthese von Leukotrienen vor (D. Pichl et al., Planta Medica, 60, 318-322 (1994)).

Nach peroraler Applikation von Petasindrogen sind in Körperflüssigkeiten gesunder Probanden lediglich Konzentrationen im Bereich von wenigen ng/ml zu erwarten. Vor diesem Hintergrund sind biologische, physikalische und chemische Nachweisverfahren, die zur Charakterisierung der Droge selbst Anwendung finden, für die Quantifizierung von Petasin in Körperflüssigkeiten nicht anwendbar. Selbst moderne analytische Methoden, wie die üblicherweise angewendete HPLC, sind nicht ausreichend empfindlich bzw. aufgrund ihres hohen Zeitaufwandes für große Probenzahlen nicht geeignet.

Der Erfindung lag deshalb die Aufgabe zugrunde Nachweismethoden für Petasin bereitzustellen, insbesondere geeignete Methoden mit hoher Sensitivität und Spezifität, die für die gewünschten pharmakokinetischen Untersuchungen eine gute Bioverfügbarkeit ermöglichen.

Erfindungsgemäß erfüllen immunchemische Detektionstechniken die gestellten Anforderungen an Sensitivität und Spezifität, wodurch eine zusätzliche, der eigentlichen Bestimmung vorangehende Extraktion bzw. Konzentrierung der Probe, wie sie bei chromatographischen Verfahren erforderlich ist, entfällt.

Die Lösung der Aufgabe konnte durch Bereitstellung von Anti-Petasin-Antikörpern, die insbesondere keine Kreuzreaktivität gegenüber Strukturanaloga oder Metaboliten des Petasins bzw. gegenüber nicht zur Immunisierung eingesetzten Derivaten des Petasins besitzen, realisiert werden.

Die erfindungsgemäßen Antikörper werden mit an ein Carrier-Molekül gekoppelten Derivaten des Petasins hergestellt. Überraschend konnte dadurch die Herstellung von Antikörpern gegen die Kopplungsgruppe des Petasins bzw. eine möglicherweise eintretende Veränderung des in der Nähe der 8-Position liegenden immundominanten Epitops vermieden werden.

Die polyklonalen oder monoklonalen Antikörper werden durch Immunisierung von nicht menschliche Säugetieren und/oder Vögeln mit Petasin oder Derivaten des Petasins der allgemeinen Formel I 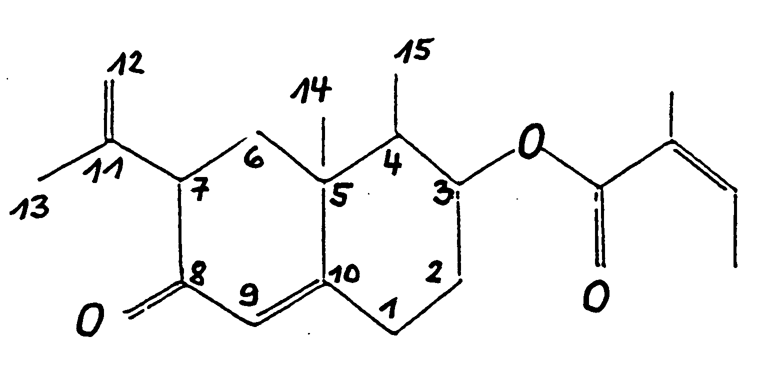 hergestellt und über Hybridomtechnik oder rekombinant mit Hilfe von Antikörperbibliotheken gewonnen.

Vorzugsweise werden folgende Carrier-Molekül-gekoppelten Derivate eingesetzt:
- Derivate des Petasins der allgemeinen Formel I, in denen die in 8-Stellung befindliche Ketogruppe durch eine carboxylgruppe, die durch Carboxymethylhydroxyamin unter Oximbildung eingeführt wird, ersetzt und mittels EDAC an Rinderserumalbumin gekoppelt ist.
- Derivate des Petasins der allgemeinen Formel I, in denen die in 8-Stellung befindliche Ketogruppe durch eine Carboxylgruppe ersetzt und über aktiviertes Hydrazid-Dextran an Rinderserumalbumin oder Fibrinogen gekoppelt ist, wobei die Einführung der Carboxylgruppe bevorzugt mit Carboxymethylhydroxyamin unter Oximbildung erfolgt.
- Derivate des Petasins der allgemeinen Formel I, in denen die in 11,12- Stellung befindliche Doppelbindung bromiert und an mittels Trautschem Reagenz aktiviertes Rinderserumalbumin gekoppelt ist.
- Derivate des Petasins der allgemeinen Formel I, in denen die Angelikasäure abgespalten ist und das verbleibende Petasol über Chlorameisensäureester an einen Träger gekoppelt ist.

Die so hergestellten Anti-Petasin-Antikörpern besitzen keine Kreuzreaktivität gegenüber, Strukturanaloga oder Metaboliten des Petasins bzw gegenüber nicht zur Immunisierung eingesetzten Derivaten des Petasins und werden zum in vitro Nachweis von Petasin oder Petasin-Protein-Konjugaten in physiologischen Flüssigkeiten eingesetzt, wobei entweder Petasin, Petasin-Protein-Konjugate oder die Anti-Petasin-Antikörper vorzugsweise mit einem Marker versehen sind. Bevorzugt liegen die Reaktionspartner in homogener Lösung vor.

Als Marker werden Enzyme, Fluoreszenzfarbstoffe, Radioisotope oder redoxaktive Verbindungen verwendet.

Das antikörpergebundene Petasin wird optisch, elektrochemisch, fluorimetrisch oder radiochemisch nachgewiesen, bevorzugt optisch mittels Farbreagenzien oder chromatographisch.

In einer Ausführungsvariante werden entweder die Anti-Petasin-Antikörper, das zu bestimmende Petasin oder die Petasin-Protein-Konjugate an eine feste Phase gebunden, wobei zwischen den Reaktionsschritten ein Waschprozeß erfolgt.

Die feste Phase ist ggf. chemisch aktiviert, wobei die Bindung der Anti-Petasin-Antikörper, des zu bestimmenden Petasins oder der Petasin-Protein-Konjugate daran adsorptiv oder kovalent erfolgt ist. Besonders bevorzugt wird als feste Phase Polystyren verwendet.

Darüber hinaus kann die feste Phase eine unterschiedliche geometrische Form aufweisen, so z.B. in Form einer Mikrotitrationsplatte, eines Röhrchens oder in kugelförmiger bzw. flächenförmiger Gestalt.

Desweiteren betrifft die Erfindung einen Testkit zur Bestimmung von Petasin in physiologischen Flüssigkeiten, der umfaßt
Anti-Petasin-Antikörper die keine Kreuzreaktivität gegenüber Strukturanaloga oder Metaboliten des Petasins, bzw. gegenüber nicht zur Immunisierung eingesetzten Derivaten des Petasins besitzen
eine feste Phase, vzw. Polystyren
Waschlösung,
Verdünnungspuffer,
markiertes Petasin oder einen markierten Anti-Species-Antikörper,
ein markerspezifisches Nachweissystem, vorzugsweise ein Enzymsubstrat.

Anschließend wird die Erfindung an Ausführungsbeisspielen näher erläutert.

### Ausführungsbeispiele

### A) Herstellung der Immunogene

### Petasinoxim:

10 mg (3,3x10⁻⁵ mol) Petasin in 5 ml Ethanol lösen, mit 15 mg (6,8x10⁻⁵ mol) Carboxymethoxylamin Hemihydrochlorid (Sigma-Aldrich) versetzen und tropfenweise 5 M Natronlauge bis zu einem pH von 12 zugeben. Der Ansatz wird 4 h am Rückfluß erhitzt, auf dem Wasserbad zur Trockne eingeengt, mit 2 M Salzsäure gewaschen und in einem Gemisch aus 1 ml Dioxan und 2 ml DMSO gelöst und bei - 70 °C gelagert.
Dünnschichtchromatografie: R_{f}-Wert (Kieselgel G60, Chloroform) = 0,42 (Petasin: 0,16).
Das Oxim entsteht als alleiniges Reaktionsprodukt.

### Petasinoxim-Rinderserumalbumin:

32 mg (4,8x10⁻⁷ mol) Rinderserumalbumin (RSA) in 4 ml PBS lösen (Lösung A).
7 mg (1,8x10⁻⁵ mol) Petasinoxim, gelöst in 1 ml Dioxan/DMSO = 1:2 (v/v), mit 16 mg 1-Ethyl-3-(3-dimethylaminopropyl)carbodiimid (EDAC) versetzen und unter Rühren 30 min bei Raumtemperatur inkubieren lassen (Lösung B).
Lösung B tropfenweise zu Lösung A geben und 6 h bei Raumtemperatur rühren, anschließend bei 4 °C gegen 3x0,5 1 PBS (0,01 M Phosphat, 0,15 M NaCl, pH=7,4) dialysieren und bei - 70 °C lagern.

### Petasin-Dextran-Proteine:

7 mg (1,8x10⁻⁵ mol) Petasinoxim, gelöst in 1 ml Dioxan/DMSO = 1:2 (v/v) tropfenweise zu 32 mg Rinderserumalbumin (4,8x10⁻⁷ mol) bzw. Fibrinogen in 4 ml PBS geben und mit 0,5 mg (1,5x10⁻⁴ mol Hydazidgruppen) aktiviertem Hydrazid-Dextran (Pierce, Code 20900) versetzen. Danach werden 16 mg 1-Ethyl-3-(3-dimethylaminopropyl)carbodiimid (EDAC) hinzugefügt und das Gemisch 4 h bei Raumtemperatur inkubiert. Anschließend wird bei 4 °C gegen 3x0,5 1 PBS (0,01 M Phosphat, 0,15 M NaCl, pH=7,4) dialysiert. Die Lagerung erfolgt bei - 70 °C.

### Brompetasin-Rinderserumalbumin:

### Bromierung von Petasin:

10 mg (3,3x10⁻⁵ mol) Petasin, gelöst in 3 ml Dichlormethan, werden tropfenweise unter Schwenken mit 5 mg (3,1x10⁻⁵ mol) Brom in 1 ml Dichlormethan versetzt. Der Ansatz wird danach im Wasserbad zur Trockne eingeengt und in 1 ml DMSO aufgenommen. Dünnschichtchromatografie: R_{f}-Wert (Kieselgel G60, Chloroform) = 0,51 (Petasin: 0,16).

### Thiolierung von Rinderserumalbumin:

40 mg (6x10⁻⁷ mol) Rinderserumalbumin in 1 ml 0,1 M Phosphatpuffer pH=8,0 lösen und mit 20 mg (1,4x10⁻⁴ mol) 2-Iminothiolan-Hydrochlorid (Traut's Reagenz) versetzen und 40 min bei Raumtemperatur inkubieren lassen. Anschließend wird mit Hilfe einer mit Sephadex G25 gefüllten Säule (1x10 cm) gegen 0,1 M Phosphatpuffer pH=7,2 umgesalzt. Zur Lösung des thiolierten Proteins gibt man unter Rühren 4 mg (8,4x10⁻⁶ mol) Brompetasin und läßt 3 h bei Raumtemperatur inkubieren, worauf bei 4 °C gegen 3x0,5 1 PBS (0,01 M Phosphat, 0,15 M NaCl, pH=7,4) dialysiert wird.

### Petasol-Rinderserumalbumin:

0,7 mg (2,9x10⁻⁶ mol) Petasol werden in 200 µl getrocknetem Dioxan/DMF = 1:1 (v/v) gelöst, mit 2 mg (7,9x10⁻⁶ mol) 5-Norbornen-2,3-dicarboximidyl-chlorkohlensäureester und 4 mg (3,3x10⁻⁵ mol) 4-Dimethylaminopyridin versetzt und unter Auschluß von Luftfeuchtigkeit 1 h bei Raumtemperatur inkubiert. Danach wird diese Lösung tropfenweise unter Rühren zu 10 mg (1,5x10⁻⁷ mol) Rinderserumalbumin, gelöst in 0,5 ml PBS, gegeben und 2 h bei Raumtemperatur inkubiert. Anschließend wird bei 4 °C gegen 3x0,5 1 PBS (0,01 M Phosphat, 0,15 M NaCl, pH=7,4) dialysiert und das Proteinkonjugat bei -70 °C gelagert.

### B) Herstellung des Antiserums

Die Immunisierung erfolgt in Kaninchen als Primärinjektion durch subkutane und intramuskuläre Injektion mit je 3 mg Petasin-RSA in komplettem Freund'schen Adjuvans. Die Sekundärinjektion erfolgt vier Wochen nach der Primärinjektion. Nach weiteren zwei Wochen erfolgt die erste Boosterinjektion, eine zweite wird zwölf Wochen nach Immunisierungsbeginn in inkomplettem Freund'schen Adjuvans durchgeführt. Ca. acht Wochen nach Beginn der Immunisierung erfolgt die erste Probeblutentnahme, die durch eine weitere nach vier Wochen ergänzt wird. Die Entblutung erfolgt nach 16 Wochen.

Die erhaltenen Antiseren werden einer Titerbestimmung auf spezifische anti-Petasin-Antikörper mittels Enzymimmunoassay unterworfen, bei dem Petasin-Ovalbumin an die Oberfläche von Mikrotiterplatten gebunden wird. Die zu untersuchenden Antiseren und Nullseren der Kaninchen werden nachfolgend in einer Verdünnungsreihe mit dem immobilisierten Petasin inkubiert. Der Nachweis der gebundenen Antikörper erfolgt durch Inkubation mit einem Ziege-anti-Kaninchen-Immunglobulin-Enzym-Konjugat (Peroxidase) und anschließender visuell auswertbarer Substratreaktion.

### C) Enzymimmunoassay

### Petasin-Ovalbumin:

0,3 mg (8x10⁻⁷ mol) Petasinoxim, gelöst in 100 µl Dioxan/DMSO = 1:2 (v/v) werden mit 4 mg EDAC versetzt und 30 min bei Raumtemperatur inkubiert. Anschließend wird der Ansatz in eine Lösung von 5,5 mg (1,2x10⁻⁷ mol) Ovalbumin in 3 ml PBS gegeben, 2 h bei Raumtemperatur unter Rühren und anschließend 16 h bei 4 °C inkubiert. Das Reaktionsgemisch wird bei 4 °C gegen 3x0,5 1 Aqua bidest. dialysiert und das Proteinkonjugat bei - 70 °C gelagert.

### Beschichtung:

Petasin-Ovalbumin wird in einer Konzentration von 5 mg/l in 0,1 M Karbonatpuffer pH=9,5 (100 µl/well) adsorptiv für 16 h bei 4 °C an Polystyren-Mikrotiterplatten gebunden und danach abgesaugt. Nach zweimaligem Waschen mit 300 µl/well Waschpuffer (PBS, 0,1% Tween 20) wird 2 h bei Raumtemperatur mit 150 µl/well Blockierungslösung (0,06% Gelatine, 0,02% Natriumazid in PBS) geblockt und schließlich dreimal mit Waschpuffer gewaschen.

### Testdurchführung:

50 µl der zu testenden Serumprobe bzw. des jeweiligen Standards (1:4-Verdünnung in Probenpuffer (PBS, 1% RSA, 0,1% Tween 20, 0,01% Thiomersal)) und 50 µl einer optimierten Antiserumverdünnung in Probenpuffer werden simultan 1 h bei Raumtemperatur unter Schütteln inkubiert. Anschließend wird die Mikrotiterplatte dreimal mit 300 µl/well Waschpuffer gewaschen und mit 100 µl anti-Kaninchen-Immunglobulin-Peroxidase-Konjugat, verdünnt in Probenpuffer, 30 min bei Raumtemperatur inkubiert und abermals wie oben gewaschen. Danach wird 10 min mit 100 µl einer gebrauchsfertigen Substratlösung (3,3',5,5'-Tetramethylbenzidin) pro Vertiefung inkubiert und die Reaktion durch Zugabe von 100 µl/well 0,5 M Schwefelsäure gestoppt. Die Auswertung erfolgt bei 450 nm in einem Mikrotiterplatten-Reader.

### Indikationsbeschreibung

Pflanzenextrakte, die mittels spezieller Verfahren aus Blätter bzw. Rhizomen von *Petasites hybridus* L. gewonnen werden, können die 5-Lipoxygenase hemmen, wodurch bei allergischen Entzündungen die Arachidonsäurekaskade wirkungsvoll unterbrochen wird. Insbesondere wird die bei Entzündungen stimulierte Leukotrienfreisetzung aus körpereigenen Zellen unterbunden, darunter auch die aus den eosinophilen und neutrophilen Leukozyten.

Somit sind derartige Pflanzenextrakte potentielle Kandidaten für die therapeutische Verwendung bei allergischen Entzündungen wie Heuschnupfen, Asthma, atopische Dermatitis, Colitis ulcerosa etc.. Erste klinische Erfahrungen belegen bereits die gute therapeutische Wirksamkeit dieses Pflanzenextraktes bei Heuschnupfen. Eine prophylaktische Verwendung des Extraktes bei ausgewählten Formen der Migraine ergab ebenfalls Hinweise für seine Wirksamkeit.

Neben der Feststellung des für die Wirksamkeit notwendigen Plasmaspiegels für relevante Inhaltsstoffe des Extraktes, beispielsweise Petasin, sind weiterhin auch die Kenntnis der Pharmakokinetik solcher relevanten Inhaltsstoffe für einen medizinischen Einsatz des Pflanzenextraktes zwingend notwendig. Mit den erfindungsgemäßen Anti-Petasin-Antikörpern in einem Enzymimmunoassay gelingt der sichere Nachweis der Petasine im Blut im unteren ng-Bereich. Das beigefügte Ergebnis einer pharmakokinetischen Untersuchung belegt eindrucksvoll die Einsatzfähigkeit.

Es wurden in einer Phase I der klinischen Prüfung zur Ermittlung pharmakokinetischer Parameter von Pestwurz-Extrakt-enthaltenden Tabletten einmalige orale Gaben von 2 bzw. 4 Tabletten an 24 klinisch gesunden Männern im Alter zwischen 18 und 40 Jahren ausgewertet.
Als Methode wurde die offene, cross-over-Prüfung gewählt, einmalige Gabe jeder Dosierung in randomisierter Reihenfolge mit einem Intervall von mindestens 7 Tagen zwischen den Gaben.

### Effektivität:

Modellunabhängige pharmakokinetische Parameter für Petasin Statistische Methoden:
ANOVA, ANOVA_{log}, Wilcoxon-Mann-Whitney-Test, Wilcoxon-Vorzeichen-Rang-Test
Zusammenfassung - Schlußfolgerungen

### Ergebnisse:

**Petasin-Serumkonzentration (ng/ml)**

| | Nach Gabe von 2 Tabletten | | | | | | Nach Gabe von 4 Tabletten | | | | | |
|---|---|---|---|---|---|---|---|---|---|---|---|---|
| Zeit p.a.(h) | N | Mean | S.D. | Min. | Median | Max. | N | Mean | S.D. | Min. | Median | Max. |
| 0 | 0 | 0,0 | | 0,0 | | 0,0 | 0 | 0,0 | | 0,0 | | 0,0 |
| 0,25 | 9 | 2,8 | 1,8 | 1,1 | 2,2 | 5,5 | 13 | 4,2 | 3,7 | 1,0 | 3,2 | 14,9 |
| 0,5 | 20 | 7,6 | 5,8 | 1,5 | 5,9 | 23,3 | 21 | 21,2 | 24,9 | 1,3 | 13,7 | 96,2 |
| 0,75 | 20 | 11,9 | 5,7 | 4,0 | 11,0 | 23,8 | 19 | 28,7 | 22,5 | 2,5 | 23,0 | 91,8 |
| 1 | 20 | 15,6 | 7,2 | 4,0 | 14,7 | 29,3 | 20 | 36,6 | 23,0 | 7,8 | 38,1 | 100,0 |
| 1,167 | 20 | 21,0 | 16,1 | 5,6 | 15,3 | 62,9 | 20 | 47,3 | 29,1 | 7,5 | 43,6 | 100,0 |
| 1,5 | 20 | 19,3 | 12,0 | 5,1 | 16,1 | 47,3 | 19 | 40,8 | 22,3 | 12,2 | 32,4 | 90,7 |
| 1,833 | 20 | 18,2 | 11,3 | 7,8 | 14,7 | 44,3 | 20 | 32,0 | 20,1 | 13,8 | 26,8 | 100,0 |
| 2,167 | 20 | 16,3 | 8,3 | 7,3 | 14,5 | 31,7 | 20 | 28,9 | 15,0 | 11,4 | 27,5 | 76,1 |
| 2,5 | 20 | 13,6 | 6,4 | 5,9 | 10,2 | 26,6 | 19 | 24,3 | 10,7 | 8,4 | 26,1 | 40,9 |
| 3 | 20 | 8,8 | 4,1 | 3,1 | 7,7 | 18,3 | 20 | 17,9 | 10,0 | 7,2 | 14,6 | 49,0 |
| 4 | 20 | 4,5 | 2,6 | 1,7 | 5,2 | 11,2 | 20 | 9,5 | 5,2 | 2,9 | 8,1 | 20,8 |
| 5 | 20 | 4,1 | 2,3 | 1,5 | 3,4 | 8,8 | 21 | 12,4 | 16,5 | 3,2 | 7,3 | 81,4 |
| 6 | 18 | 3,2 | 1,7 | 1,2 | 3,1 | 8,1 | 21 | 5,8 | 3,8 | 1,6 | 5,0 | 14,7 |
| 8 | 18 | 1,9 | 0,8 | 1,0 | 1,6 | 4,2 | 19 | 3,9 | 3,1 | 1,4 | 3,1 | 15,7 |
| 12 | 13 | 1,6 | 0,5 | 1,1 | 1,4 | 2,9 | 18 | 2,7 | 1,0 | 1,3 | 2,5 | 5,1 |
| 24 | 6 | 1,5 | 0,5 | 1,1 | 1,3 | 2,3 | 10 | 1,3 | 0,4 | 1,0 | 1,0 | 2,3 |

Werte unter der Bestimmungsgrenze (1 ng/ml) gleich 0 gesetzt

**Modellunabhängige pharmakokinetische Parameter (± S.D.)**

| Parameter/Dosierung | 2 Tabletten | 4 Tabletten |
|---|---|---|
| Cₘₐₓ (ng/ml) | 25,5 | 58,1 |
| SD | ±14,8 | ± 26,7 |
| tₘₐₓ (h) | 1,616 | 1,614 |
| SD | ±0,499 | ± 0,926 |
| AUC₀₋ₜ₍ₗₐₛₜ₎ (ng/ml*h) | 65,30 | 151,15 |
| SD | ± 35,61 | ± 68,21 |
| AUC_{0-∞} (ng/ml*h) | 79,68 | 168,22 |
| SD | ± 42,27 | ± 73,43 |
| AUC_{Rest} (%) | 18,3 | 10,8 |
| SD | ± 7,9 | ± 4,9 |
| T_{1/2} (h) | 7,155 | 7,618 |
| SD | ± 4,611 | ± 3,338 |
| MRT (h) | 7,32 | 6,74 |
| SD | ± 3,74 | ± 2,47 |

### Sicherheitsparameter:

Keine signifikanten und klinisch relevanten Veränderungen der hämatologischen und klinisch-chemischen Laborparameter.

### Unerwünschte Ereignisse:

Unerwünschte Ereignisse traten nicht auf.

### Schlußfolgerungen:

- Die Resorption erfolgt rasch und dosisabhängig.
- Beide Dosierungen sind hinsichtlich ihrer Bioverfügbarkeit als gleich anzusehen.

### Mathematisch-statistische Auswertung

### 1. Pharmakokinetische und statistische Berechnungen

Basis der Auswertung waren die gemessenen Serumspiegel von Petasin

### 2. Modellunabhängige pharmakokinetische Parameter

Die Mittelwerte und Standardabweichungen (SD) der pharmakokinetischen Parameter sind in der Tabelle 1 zusammengefaßt

| Parameter/Dosierung | 2 Tabletten | 4 Tabletten |
|---|---|---|
| Cₘₐₓ (ng/ml) ± SD | 25,5 ± 14,8 | 58,1 ± 26,7 |
| tₘₐₓ (h) ± SD | 1,616 ±0,499 | 1,614 ± 0,926 |
| AUC₀₋ₜ₍ₗₐₛₜ₎ (ng/ml*h) ± SD | 65,30 ± 35,61 | 151,15 ± 68,21 |
| AUC_{0-∞} (ng/ml*h) ± SD | 79,68 ± 42,27 | 168,22 ± 73,43 |
| AUC_{Rest} (%) ± SD | 18.3 ± 7,9 | 10,8 ± 4,9 |
| t_{1/2} (h) ± SD | 7,155 ± 4,611 | 7,618 ± 3,338 |
| MRT (h) ± SD | 7,32 ± 3,74 | 6,74 ± 2,47 |

Die dosisabhängigen Parameter Cₘₐₓ und AUC verhalten sich nahezu dosisproportional, die Abweichungen der Mittelwerte aller anderen Parameter sind, unter Berücksichtigung der ermittelten Standardabweichungen nahezu identisch.
Die großen Standardabweichungen sind als Ausdruck interindividueller Unterschiede vor allem in der Geschwindigkeit der Resorption, Verteilung und des Matabolismus von Petasin zu betrachten. So wurde bei einem Probanden nach Gabe der niedrigen Dosierung zu keinem Zeitpunkt ein Petasinserumspiegel über der Bestimmungsgrenze der Analysenmethode gefunden.

Die Berechnung der relevanten Bioverfügbarkeit (Berechnung der dosiskorrigierten Quotienten der pharmakokinetischen Parameter mit 90 % Konfidenzintervall) der Gabe von 4 Tabletten im Vergleich zur Gabe von 2 Tabletten der Prüfmedikation ergibt:

**Tabelle 2 Relative Bioverfügbarkeit Pestwurz 4 Tabletten versus Pestwurz 2 Tabletten**

| | | Cₘₐₓ | T/R (%) | AUC_{0-∞} | T/R (%) |
|---|---|---|---|---|---|
| Verteilungs-Annahme | Statistische Methode | Punkt-Schätz. | Konf.interv. von ... bis | Punkt-Schätz. | Konf.interv. von ... bis |
| Normalvert. | ANOVA (x-Over) | 113,5 | 91,9 ... 135,0 | 106,2 | 86,5 ... 126,0 |
| log-Normalvert. | ANOVA log (x-Over) | 114,9 | 94,7 ... 139,5 | 109,1 | 92,5 ... 128,6 |
| Verteilungsfrei | Wilcoxon-Mann-Whitney Test | 113,5 | 87,7 ... 141,8 | 101,0 | 87,5 ... 121,3 |
| | Wilcoxons Vorzeichen-Rang-Test | 111,4 | 93,4 ... 135,4 | 104,5 | 90,8 ... 122,3 |

Im Rahmen der bei Bioäquivalenzprüfungen gewöhnlich akzeptierten Grenzen von 70 bis 142,9 % für Cₘₐₓ und von 80 bis 125 % für AUC ist die Verfügbarkeit beider Dosierungen als gleich anzusehen.

**Tabelle 3 Gruppenstatistiken der Petasinkonzentration (ng/ml) im Serum nach Gabe von 4 Tabletten**

| Zeit p.a. | N | Mean | S.D. | Min. | Median | Max. |
|---|---|---|---|---|---|---|
| 0 | 0 | <1 | | | <1 | |
| 0,25 | 13 | 4,2 | 3,7 | 1,0 | 3,2 | 14,9 |
| 0,5 | 21 | 21,2 | 24,9 | 1,3 | 13,7 | 96,2 |
| 0,75 | 19 | 28,7 | 22,5 | 2,5 | 23,0 | 91,8 |
| 1 | 20 | 36,6 | 23,0 | 7,8 | 38,1 | 100,0 |
| 1,167 | 20 | 47,3 | 29,1 | 7,5 | 43,6 | 100,0 |
| 1,5 | 19 | 40,8 | 22,3 | 12,2 | 32,4 | 90,7 |
| 1,833 | 20 | 32,0 | 20,1 | 13,8 | 26,8 | 100,0 |
| 2,167 | 20 | 23,9 | 15,0 | 11,4 | 27,5 | 76,1 |
| 2,5 | 19 | 24,3 | 10,7 | 8,4 | 26,1 | 40,9 |
| 3 | 20 | 17,9 | 10,0 | 7,2 | 14,6 | 49,0 |
| 4 | 20 | 9,5 | 5,2 | 2,9 | 8,1 | 20,8 |
| 5 | 21 | 12,4 | 16,5 | 3,2 | 7,3 | 81,4 |
| 6 | 21 | 5,8 | 3,8 | 1,6 | 5,0 | 14,7 |
| 8 | 19 | 3,9 | 3,1 | 1,4 | 3,1 | 15,7 |
| 12 | 18 | 2,7 | 1,0 | 1,3 | 2,5 | 5,1 |
| 24 | 10 | 1,3 | 0,4 | 1,0 | 1,0 | 2,3 |

Werte unter der Bestimmungsgrenze (1 ng/ml) gleich 0

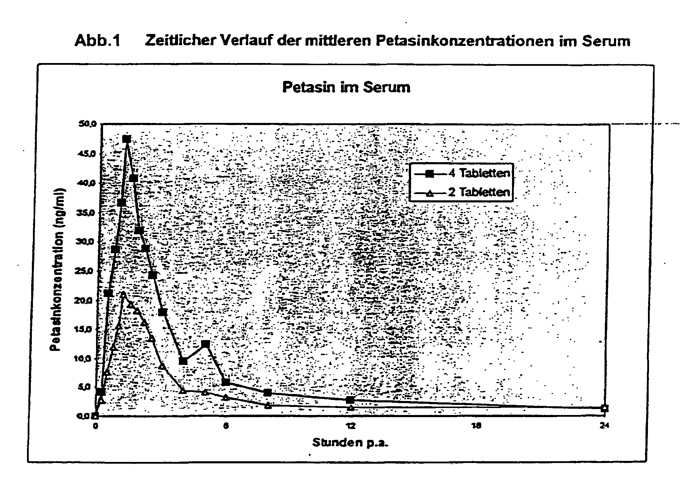

Aus Abb.1 ist ersichtlich, daß sich die mittlere maximale Petasinkonzentration (Cₘₐₓ) nach Gabe der doppelten Dosis annähernd verdoppelt. Der mittlere Zeitpunkt des Erreichens maximaler Serumspiegel(tₘₐₓ) bleibt konstant.

## Patentansprüche

1. Anti-Petasin-Antikörper zum in vitro Nachweis von Petasin oder Petasin-Protein-Konjugaten in physiologischen Flüssigkeiten, die keine Kreuzreaktivität gegenüber Strukturanaloge oder Metaboliten des Petasins bzw. gegenüber nicht zur Immunisierung eingesetzten Derivaten des Petasins besitzen.

2. Verfahren zur Herstellung von Anti-Petasin-Antikörpern, **dadurch gekennzeichnet, daß** polyklonale oder monoklonale Antikörper durch Immunisierung von nichtmenschlichen Säugetieren und/oder Vögeln mit Petasin oder Petasin-Derivaten der allgemeinen Formel I 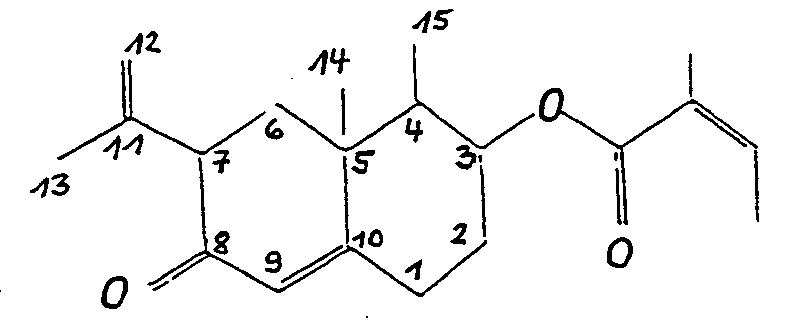 hergestellt und die Antikörper über Hybridomtechnik oder rekombinant mit Hilfe von Antikörperbibliotheken gewonnen werden, wobei
als Petasin-Derivate Carrier-Molekül-gekoppelte Derivate eingesetzt werden.

3. Verfahren nach Anspruch 2, **dadurch gekennzeichnet, daß** zur Immunisierung Derivate des Petasins eingesetzt werden, in denen die in 8-Stellung befindliche Ketogruppe durch eine Carboxylgruppe ersetzt und mittels EDAC an Rinderserumalbumin gekoppelt ist.

4. Verfahren nach Anspruch 2, **dadurch gekennzeichnet, daß** zur Immunisierung Derivate des Petasins eingesetzt werden, in denen die in 8-Stellung befindliche Ketogruppe durch eine Carboxylgruppe ersetzt und über aktiviertes Hydrazid-Dextran an Rinderserumalbumin oder Fibrinogen gekoppelt ist.

5. Verfahren nach Anspruch 3 und 4, **dadurch gekennzeichnet, daß** die Einführung der Carboxylgruppe mit Carboxymethylhydroxyamin unter Oximbildung erfolgt.

6. Verfahren nach Anspruch 2, **dadurch gekennzeichnet, daß** zur Immunisierung Derivate des Petasins eingesetzt werden, in denen die in 11,12- Stellung befindliche Doppelbindung bromiert und an mittels Trautschem Reagenz aktiviertes Rinderserumalbumin gekoppelt ist.

7. Verfahren nach Anspruch 2, **dadurch gekennzeichnet, daß** zur Immunisierung Derivate des Petasins eingesetzt werden, in denen die Angelikasäure abgespalten ist und das verbleibende Petasol über Chlorameisensäureester an einen Carrier gekoppelt ist.

8. Verwendung von Anti-Petasin-Antikörpern gemäß Anspruch 1 zum in vitro Nachweis von Petasin oder Petasin-Protein-Konjugaten in physiologischen Flüssigkeiten.

9. Verwendung nach Anspruch 8, **dadurch gekennzeichnet, daß** entweder Petasin, Petasin-Protein-Konjugate oder die Anti-Petasin-Antikörper mit einem Marker versehen sind.

10. Verwendung nach Anspruch 9, **dadurch gekennzeichnet, daß** Marker Enzyme, Fluoreszenzfarbstoffe, Radioisotope oder redoxaktive Verbindungen sind.

11. Verwendung nach einem der Ansprüche 8 bis 10, **dadurch gekennzeichnet, daß** der Nachweis antikörpergebundenen Petasins optisch, elektrochemisch, fluorimetrisch oder radiochemisch erfolgt.

12. Verwendung nach Anspruch 11, **dadurch gekennzeichnet, daß** ein Farbreagenz verwendet wird.

13. Verwendung nach Anspruch 11, **dadurch gekennzeichnet, daß** der Nachweis chromatographisch erfolgt.

14. Verwendung nach einem der Ansprüche 8 bis 13, **dadurch gekennzeichnet, daß** die Reaktionspartner in homologer Lösung vorliegen.

15. Verwendung nach einem der Ansprüche 8 bis 14, **dadurch gekennzeichnet, daß** entweder die Anti-Petasin-Antikörper, das zu bestimmende Petasin oder die Petasin-Protein-Konjugate an einer feste Phase gebunden sind und zwischen den Reaktionsschritten ein Waschprozeß erfolgt.

16. Verwendung nach Anspruch 15, **dadurch gekennzeichnet, daß** die Bindung der Anti-Petasin-Antikörper, des zu bestimmenden Petasins oder der Petasin-Protein-Konjugate an die feste Phase adsorptiv oder nach vorhergehender chemischer Aktivierung der festen Phase kovalent erfolgt.

17. Verwendung nach Anspruch 15 oder 16, **dadurch gekennzeichnet, daß** die feste Phase aus Polystyren besteht.

18. Verwendung nach einem der Ansprüche 15 bis 17, **dadurch gekennzeichnet, daß** die feste Phase eine unterschiedliche geometrische Form hat.

19. Verwendung nach Anspruch 18, **dadurch gekennzeichnet, daß** sie in Form einer Mikrotitrationsplatte, eines Röhrchens oder kugelförmige bzw. flächenförmige Gestalt aufweist.

20. Testkit zur Bestimmung von Petasin in physiologischen Flüssigkeiten umfassend
Anti-Petasin-Antikörper gemäss Anspruch 1
eine feste Phase, vzw. Polystyren
Waschlösung,
Verdünnungspuffer,
Enzymmarkiertes Petasin.

## Claims

1. Anti-petasin antibodies for in vitro detection of petasin or petasin protein conjugates in physiological fluids not possessing cross-reactivity against structural analogues or metabolites of petasin or against derivates of petasin not used for immunisation.

2. Method for the production of anti-petasin antibodies, wherein polyclonal or monoclonal antibodies are produced by immunisation of non-human mammals and/or birds with petasin or petasin derivates of the general formula I 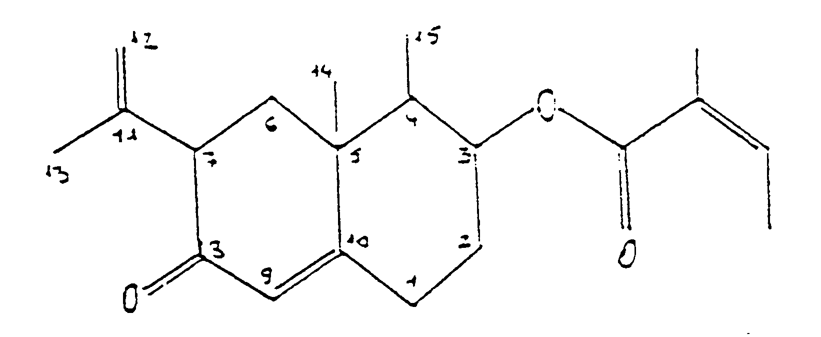 and the antibodies are obtained by hybridoma technique or recombinantly with the help of antibody libraries, with carrier molecule coupled derivates being used as petasin derivates.

3. Method according to Claim 2, wherein derivates of the petasin in which the keto group to be found in position 8 has been replaced by a carboxyl group and coupled to cattle serum albumin by means of EDAC are used for immunisation.

4. Method according to Claim 2, wherein derivates of the petasin in which the keto group to be found in position 8 has been replaced by a carboxyl group and coupled to cattle serum albumin or fibrinogen via activated hydracide-dextran are used for immunisation.

5. Method according to Claim 3 and 4, wherein the insertion of the carboxyl group is done with carboxymethylhydroxyamine with oxim formation.

6. Method according to Claim 2, wherein derivates of the petasin in which the double binding to be found in position 11,12 has been bromated and coupled to activated cattle serum albumin by means of Traut's reagent are used for immunisation.

7. Method according to Claim 2, wherein derivates of the petasin in which the angelic acid has been fissured and the remaining petasol has been coupled to a carrier by means of chloroformate are used for immunisation.

8. Use of anti-petasin antibodies according to Claim 1 for the in vitro detection of petasin or petasin protein conjugates in physiological fluids.

9. Use according to Claim 8, wherein either petasin, petasin protein conjugates or the anti-petasin antibodies have been provided with a marker.

10. Use according to Claim 9, wherein markers are enzymes, fluorescence dyes, radioisotopes or redox active compounds.

11. Use according to one of the claims 8 to 10, wherein the detection of antibody-bound petasin is done visually, electrochemically, fluorimetrically or radiochemically.

12. Use according to Claim 11, wherein a colour reagent is used.

13. Use according to Claim 11, wherein the detection is done chromatographically.

14. Use according to one of the claims 8 to 13, wherein the reaction partners are available in a homologous solution.

15. Use according to one of the claims 8 to 14, wherein either the anti-petasin antibodies, the petasin to be determined or the petasin protein conjugates have been bound to a firm phase and a washing process takes place between the reaction steps.

16. Use according to Claim 15, wherein the binding of the anti-petasin antibodies, the petasin to be determined or the petasin protein conjugates to the firm phase is done adsorptively or covalently following the prior chemical activation of the firm phase.

17. Use according to Claim 15 or 16, wherein the firm phase comprises polystyrene.

18. Use according to one of the claims 15 to 17, wherein the firm phase has a differing geometric shape.

19. Use according to Claim 18, wherein it manifests the form of a micro-titration plate, a tube or a spherical or planiform design.

20. Test kit for the determination of petasin in physiological fluids, comprising
anti-petasin antibodies according to Claim 1
a firm phase, preferably polystyrene
washing solution,
dilution buffer,
enzyme-marked petasin.

## Revendications

1. Anticorps anti-pétasine pour la mise en évidence in vitro de la pétasine ou de conjugué de pétasine et de protéine dans des liquides physiologiques qui ne possèdent aucune réactivité croisée par rapport aux analogues structurels ou aux métabolites de la pétasine voire par rapport à des dérivés de la pétasine utilisés pour l'immunisation.

2. Procédé de fabrication d'anticorps anti-pétasine, **se caractérisant par le fait que** des anticorps polyclonaux et monoclonaux sont fabriqués par immunisation de mammifères non humains et/ou d'oiseaux avec de la pétasine ou des dérivés de pétasine de la formule générale 1 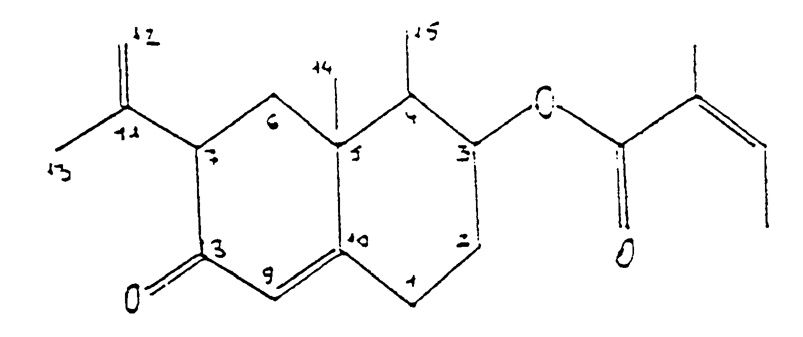 et les anticorps sont extraits par la technique des hybridomes ou en recombinaison avec l'aide des bibliothèques d'anticorps, en utilisant des dérivés couplés à une molécule porteuse en tant que dérivés de la pétasine.

3. Procédé selon la revendication 2, **se caractérisant par le fait que** des dérivés de la pétasine sont utilisés pour l'immunisation, dérivés dans lesquels le carbonyle se trouvant dans la position 8 est remplacé par un groupe carboxyle et est couplé a l'albumine de sérum de boeuf au moyen de EDAC.

4. Procédé selon la revendication 2, **se caractérisant par le fait que** des dérivés de la pétasine sont utilisés pour l'immunisation, dérivés dans lesquels le carbonyle se trouvant dans la position 8 est remplacé par un groupe carboxyle et est couplé à l'albumine de sérum de boeuf ou au fibrinogène au moyen du dextrane d'hydrazide.

5. Procédé conformément à la revendication 3 et 4, **se caractérisant par le fait que** l'introduction du groupe carboxyle se fait avec de la carboxymethylhydroxyamine avec la formation d'oxime.

6. Procédé selon la revendication 2, **se caractérisant par le fait que** des dérivés de la pétasine sont utilisés pour l'immunisation, dérivés dans lesquels la double liaison se trouvant dans la position 11,12 est bromée et couplée à l'albumine de sérum de boeuf activée au moyen du réactif Trautsche.

7. Procédé selon la revendication 2, **se caractérisant par le fait que** des dérivés de la pétasine sont utilisés pour l'immunisation, dérivés dans lesquels l'acide angélique est séparé et le Petasol restant est couplé à un porteur par l'ester de l'acide chloroformique.

8. Emploi d'anticorps anti-pétasine conformément à la revendication 1 pour mettre en évidence in vitro de la pétasine ou des conjugués de pétasine et de protéine dans des liquides physiologiques.

9. Emploi selon la revendication 8, **se caractérisant par le fait que** ni la pétasine, ni les conjugués de pétasine et de protéine ou les anticorps anti-pétasine sont munis d'un marqueur.

10. Emploi selon la revendication 9, **se caractérisant par le fait que** les marqueurs sont des enzymes, des colorants fluorescents, des radio-isotopes ou des liaisons d'oxydoréduction.

11. Emploi selon l'une des revendications 8 à 10, **se caractérisant par le fait que** la mise en évidence de pétasine liée à l'anticorps se fait de façon optique, électrochimique, fluorimétrique ou radiochimique.

12. Emploi selon la revendication 11, **se caractérisant par le fait que** l'on utilise un réactif colorant.

13. Emploi selon la revendication 11, **se caractérisant par le fait que** la mise en évidence se fait de façon chromatographique.

14. Emploi selon l'une des revendications 8 à 13, **se caractérisant par le fait que** le partenaire réactif se trouve dans une solution homologue.

15. Emploi selon l'une des revendications 8 à 14, **se caractérisant par le fait que** soit les anticorps anti-pétasine, soit la pétasine à déterminer ou les conjugués de pétasine et de protéine sont liés à une phase solide et qu'un processus de lavage se fait entre les étapes de la réaction.

16. Emploi selon la revendication 15, **se caractérisant par le fait que** la liaison des anticorps anti-pétasine, de la pétasine à déterminer ou des conjugués de pétasine et de protéine se fait par adsorption ou par covalence après activation chimique préalable de la phase solide.

17. Emploi selon la revendication 15 ou 16, **se caractérisant par le fait que** la phase solide se compose de polystyrène.

18. Emploi selon l'une des revendications 15 à 17, **se caractérisant par le fait que** la phase solide a une forme géométrique différente.

19. Emploi selon la revendication 18, **se caractérisant par le fait qu'**elle se présente sous forme d'une plaquette de microtitration, d'une éprouvette ou d'une structure sphérique voire planiforme.

20. Kit de test pour déterminer la pétasine dans des liquides physiologiques englobant
des anticorps anti-pétasine conformément à la revendication 1
d'une phase solide, voire de polystyrène
d'une solution de lavage,
d'un tampon de dilution,
de la pétasine marquée aux enzymes.
